Europäisches Patentamt

European Patent Office

Office européen des brevets

(11) Numéro de publication: **0 355 075 B1**

(12) **FASCICULE DE BREVET EUROPEEN**

(45) Date de publication de fascicule du brevet: **08.06.94**

(51) Int. Cl.5: **C07C 51/31**, C07C 55/14,
C07C 67/42, C07C 69/716,
C07C 51/245, C07C 59/185

(21) Numéro de dépôt: 89420303.3

(22) Date de dépôt: **10.08.89**

(54) **Procédé de préparation d'acides carboxyliques aliphatiques.**

(30) Priorité: **16.08.88 FR 8811075**

(43) Date de publication de la demande:
**21.02.90 Bulletin 90/08**

(45) Mention de la délivrance du brevet:
**08.06.94 Bulletin 94/23**

(84) Etats contractants désignés:
**BE DE ES FR GB IT NL**

(56) Documents cités:
EP-A- 0 063 955
GB-A- 999 229
US-A- 3 590 080

(73) Titulaire: **RHONE-POULENC CHIMIE**
**25, quai Paul Doumer**
**F-92408 Courbevoie Cédex(FR)**

(72) Inventeur: **Bregeault, Jean-Marie**
**7, rue des Saules**
**F-91800 Boussy St-Antoine(FR)**
Inventeur: **El-Ali, Bassam**
**Université Pierre et Marie Curie**
**Place Jussieu**
**F-75252 Paris Cedex 05(FR)**
Inventeur: **Martin, Jacques**
**Résidence de Chevreuse B1**
**34, Avenue St-Laurent**
**F-91400 Orsay(FR)**

(74) Mandataire: **Vignally, Noel et al**
**Rhône-Poulenc Chimie**
**Direction de la Propriété Industrielle**
**Centre de Recherches des Carrières**
**B.P. 62**
**F-69192 Saint-Fons Cédex (FR)**

## Description

La présente invention concerne un procédé de préparation d'acides carboxyliques aliphatiques par oxydation de cétones cycliques au moyen d'oxygène moléculaire ou d'un gaz en contenant.

Le brevet GB-A-999229 décrit un procédé d'oxydation de composés tels que la cyclohexanone, le cyclohexylhydropéroxyde ou la cyclohexanedione-1,2 à l'aide d'une quantité au moins stoechiométrique d'un composé pentavalent du vanadium. Ce composé de vanadium est ainsi transformé en composé tétravalent du vanadium qui est lui-même réoxydé par l'acide nitrique en composé pentavalent.

La présente invention a plus spécifiquement pour objet un procédé de préparation d'acides carboxyliques aliphatiques par oxydation de cétones monocyliques au moyen d'oxygène moléculaire ou d'un gaz en contenant, caractérisé en ce que le catalyseur est choisi parmi les composés du vanadium répondant à l'une quelconque des formules (I) et (II) ci-après :

$$H_{(3+n)} [PM_{12-n} V_n O_{40}], y\ H_2O \qquad (I)$$

$$VO(Y)_m \qquad (II)$$

dans lesquelles :
- n est un entier supérieur ou égal à 1 et inférieur ou égal à 6,
- M représente un atome de molybdène ou de tungstène,
- y est un entier, pouvant être nul, inférieur à 50,
- Y représente un groupe acétylacétonate ou un groupement alcoxy comportant de 1 à 10 atomes,
- m a pour valeur 2 ou 3.

Par cétones monocycliques, matières de départ, dans le cadre du présent procédé on entend des composés dont le groupe carbonyle est lié directement à des atomes de carbone pour former un cycle unique saturé de 5 à 8 atomes de carbone, le cycle pouvant comporter en outre un ou deux substituants choisis parmi les radicaux alkyles en $C_1$-$C_4$ et le groupe phényle.

A titre d'exemples de telles cétones on peut citer :
- la cyclopentanone,
- la cyclohexanone,
- la méthyl-2 cyclohexanone,
- la méthyl-2 cyclopentanone,
- la diméthyl-2,5 cyclopentanone
- la diméthyl-2,6 cyclohexanone et
- la phényl-2 cyclohexanone.

Par acides carboxyliques aliphatiques on entend des cétoacides et des diacides. Ces acides sont susceptibles d'être au moins partiellement convertis, in situ en présence d'un alcool ou d'un éther, en esters ou diesters correspondants, dès leur formation. La préparation de ces formes partiellement ou totalement estérifiées des acides en cause entre dans le cadre du présent procédé.

La cyclohexanone est une matière de départ particulièrement préférée, car elle conduit à l'acide adipique.

Le procédé selon la présente invention requiert la mise en oeuvre d'un catalyseur choisi parmi les composés du vanadium répondant à l'une quelconque des formules (I) et (II) définies ci-avant.

A titre d'exemples de composés du vanadium répondant à la formule (I) on peut citer :

$H_4\{PW_{11}V_1O_{40}\}$, 30 $H_2O$
$H_4\{PMo_{11}V_1O_{40}\}$, 33 $H_2O$
$H_5\{PMo_{10}V_2O_{40}\}$, 30-36 $H_2O$
$H_6\{PMo_9V_3O_{40}\}$, 34 $H_2O$

A titre d'exemples de composés du vanadium répondant à la formule (II) on peut citer :
- le bis(acétylacétonate) de vanadyle et
- le vanadate d'isopropyle.

Dans le cadre du présent procédé on préfère recourir à des composés du vanadium répondant à la formule (I) ci-avant.

Plus spécifiquement on recourt à des composés répondant à la formule (I) dans laquelle :
- n vaut 1 ou 2 et
- y est supérieur ou égal à 30 et inférieur ou égal à 40.

Une classe avantageuse de catalyseurs à base de vanadium est constituée par les composés répondant à la formule (I) indiquée en tête du présent mémoire, dans laquelle M est un atome de molybdène.

Ces composés (acides molybdo- ou tungstovanadophosphoriques) ainsi que leurs modes de préparation sont connus de l'homme de l'art. On pourra se reporter utilement à l'article de Messieurs G.A. TSIGDINOS et C.J. HALLADA paru dans Inorganic Chemistry, Vol. 7, mars 1968, pages 437 et suivantes, à celui de P. COURTIN, publié dans Rev. Chim. Min., 1971, 8, 75 et à celui de M. CANNERI, dans Gazz. Chim. Ital., 1926, 56, 871.

La quantité de catalyseur à mettre en oeuvre peut varier dans de larges limites. Cette quantité exprimée en mole de vanadium par litre de milieu réactionnel est généralement comprise entre 0,001 et 0,5 mol-l$^{-1}$. De préférence, cette concentration est comprise entre 0,005 et 0,1 mol.l$^{-1}$.

L'oxydation a généralement lieu en milieu liquide, le milieu liquide comprenant la cétone monocyclique à oxyder et, le cas échéant, un solvant ou diluant inerte dans les conditions réactionnelles, vis-à-vis des constituants du mélange réactionnel.

A titres d'exemples de tels solvants ou diluants, on peut citer les hydrocarbures aromatiques halogénés ou non, les hydrocarbures nitrés tels le nitrobenzène et le nitrométhane, les nitriles tel l'acétonitrile et des acides carboxyliques tels l'acide acétique et, l'eau.

L'oxydation peut également être conduite en milieu liquide, ledit milieu comprenant un alcool et, en particulier un alcanol en $C_1$-$C_4$ qui est susceptible de réagir avec l'acide (ou le diacide) produit pour former l'ester (ou diester) correspondant.

Bien entendu, le milieu réactionnel peut renfermer un mélange de solvant et, en particulier un mélange de solvant inerte au sens indiqué ci-avant et d'un alcool.

Le milieu réactionnel peut également renfermer un éther tel le diglyme.

La concentration initiale du substrat à oxyder peut varier dans de larges limites. En général elle se situe entre 50 et 400 g.l$^{-1}$, de préférence entre 150 et 250 g.l$^{-1}$.

La température à laquelle est mis en oeuvre le procédé selon la présente invention est habituellement comprise entre 25 et 120 °C et se situe de préférence entre 40 et 100 °C.

L'oxygène moléculaire utilisé comme agent oxydant dans le présent procédé peut être sous forme d'oxygène pur ou de mélanges d'oxygène avec d'autres gaz inertes. On peut ainsi employer l'air ou encore des mélanges oxygène-azote plus riches ou moins riches en oxygène que l'air.

L'oxydation peut être effectuée sous courant d'oxygène ou du gaz contenant de l'oxygène ou par charge d'une certaine quantité d'oxygène. La pression partielle d'oxygène peut varier entre 0,2 bar et 20 bars ; mais en pratique il n'est pas nécessaire qu'elle dépasse 10 bars.

En fin de réaction ou du temps de réaction souhaité, on peut récupérer et séparer les produits obtenus par tout moyen approprié, par exemple par distillation.

Les exemples ci-après illustrent la présente invention.

- <u>EXEMPLES 1 A 5</u> :

Ces exemples illustrent l'oxydation de la méthyl-2 cyclohexanone.

Dans un tube de verre de 20 ml, relié à un gazomètre contenant de l'oxygène moléculaire, on place 12,4 mmol de méthyl-2 cyclohexanone, le catalyseur dont la nature et la quantité sont indiquées au tableau ci-après, pour les exemples 2 à 5, 6 cm$^3$ d'acétonitrile.

Après fermeture du tube, on le porte à 60 °C sous agitation pendant la durée indiquée au tableau.

Au bout de 4 heures (ou 24 heures) en température, le mélange est refroidi et analysé par chromatographie en phase vapeur.

Les conditions particulières ainsi que les résultats obtenus figurent au tableau (I) ci-après dans lequel :
- S (AOH) désigne la sélectivité en acide oxo-6 heptanoïque,
-

$$\left[ \frac{\text{nombre de moles de produit formé}}{\text{nombre de moles de substrat transformé}} \times 100 \right]$$

- TT désigne le taux de transformation de la méthyl-2 cyclohexanone.

TABLEAU I

| EX n° | CATALYSEUR | | Temps | TT | S(AOH) |
|---|---|---|---|---|---|
| | NATURE | mol.l$^{-1}$ | (h) | (%) | (%) |
| 1 | VO(acac)$_2$ | 0,12 | 24 | 49 | 89,0 |
| 2 | VO{OCH(CH$_3$)$_2$}$_3$ | 0,02 | 24 | 57,5 | 89,5 |
| 3 | H$_4${PW$_{11}$V$_1$O$_{40}$}, 30 H$_2$O | 0,02 | 4 | 57 | 89,5 |
| 4 | H$_4${PMo$_{11}$V$_1$O$_{40}$}, 33 H$_2$O | 0,02 | 4 | 93,5 | 89,5 |
| 5 | H$_5${PMo$_{10}$V$_2$O$_{40}$}, 30-36 H$_2$O | 0,01 | 4 | 98 | 89,0 |

- EXEMPLE 6 :

Cet exemple illustre l'oxydation de la cyclohexanone.

On opère de manière analogue à l'exemple 5 ci-avant en remplaçant la méthyl-2 cyclohexanone par la même quantité molaire de cyclohexanone.

Toutes conditions égales par ailleurs on a obtenu de l'acide adipique avec une sélectivité de 75 %, le taux de transformation de la cyclohexanone étant de 85 %.

- EXEMPLE 7 :

On reproduit l'exemple 5 ci-avant en remplaçant l'acétonitrile par le même volume de nitrométhane. Toutes conditions égales par ailleurs on a obtenu les résultats suivants :

Le taux de transformation de la méthyl-2 cyclohexane :

(TT) est de 98 %

Le rendement en acide oxo-6 heptanoïque {RR (AOH) } est de 81,5 %

$$\left[ RR = \frac{\text{nombre de moles de produit formé}}{\text{nombre de moles de substrat initial}} \times 100 \right]$$

- EXEMPLE 8 :

On reproduit l'exemple 5 ci-avant en remplaçant l'acétonitrile par le même volume d'acide acétique.

En 6 heures de réaction toutes conditions égales par ailleurs on a obtenu les résultats suivants :

TT = 81 %

RR (AOH) = 69 %

- EXEMPLE 9 :

On reproduit l'exemple 5 ci-avant en remplaçant l'acétonitrile par le même volume de méthanol.

En 6 heures de réaction, toutes conditions égales par ailleurs on a obtenu les résultats suivants :

TT = 54%

Le rendement en ester méthylique de l'acide oxo-6 heptanoöque est de 49 % {RR(EST) }

- EXEMPLE 10 :

On reproduit l'exemple 5 ci-avant en remplaçant l'acétonitrile par le même volume de diglyme (CH$_3$-O-CH$_2$-CH$_2$-O-CH$_3$).

En 24 heures de réaction, toutes conditions égales par ailleurs on a obtenu les résultats suivants :

TT = 96 %

RR(AOH) = 74 %
RR(EST) = 16 %

- EXEMPLE 11 :

On reproduit l'exemple 5 ci-avant en remplaçant 1 cm3 d'acétonitrile par 1 cm3 de méthanol.
En 6 heures de réaction toutes conditions égales par ailleurs on a obtenu les résultats suivants :
TT = 96 %
RR(AOH) = 4 %
RR(EST) = 86 %

- EXEMPLES 12 A 14 :

Selon le mode opératoire décrit pour les exemples 1 à 5 ci-avant on réalise une série d'essais sur une charge ne renfermant par de solvant mais 12,4 mmol (1,5 cm3) de méthyl-2 cyclohexanone, un catalyseur dont la nature et la quantité figurent au tableau (II) ci-après, à 60°C sous une pression partielle d'oxygène d'une atmosphère. Les conditions particulières et les résultats obtenus figurent dans le tableau (II) ci-après dans lequel les conventions utilisées sont les suivantes :
-   TT désigne le taux de transformation de la méthyl-2 cyclohexanone.
-   RR (AOH) désigne le rendement en acide oxo-6 heptanoïque

$$RR = \frac{\text{nombre de moles de produit formé}}{\text{nombre de moles de substrat initial}} \times 100$$

TABLEAU II

| EX. N° | CATALYSEUR | | DUREE (h) | TT (%) | TT(AOH) (%) |
|---|---|---|---|---|---|
| | NATURE | mmol | | | |
| 12 | $H_5\{PMo_{10}V_2O_{40}\}$, 30-36$H_2O$ | 0,075 | 8 | 90 | 75 |
| 13 | $VO\{OCH(CH_3)_2\}_3$ | 0,375 | 24 | 50 | 40 |
| 14 | $\{VO(acac)_2\}$ | 0,90 | 24 | 49 | 41 |

- EXEMPLE 15 :

Selon le mode opératoire décrit précédemment on réalise un essai sur une charge renfermant :
-   4,85 mmol de cyclohexanone
-   5 cm3 d'acétonitrile
-   1 cm3 de méthanol
-   0,05 mmol de $H_5\{PMo_{10}V_2O_{40}\}$, 30-36$H_2O$
La pression partielle d'oxygène est de 1 atm. En 24 heures de réaction à 60°C on a obtenu les résultats suivants :
TT = 98 %
Rendement en adipate de diméthyle : 54 %.

- EXEMPLE 16 à 20 :

Selon le mode opératoire décrit précédemment on réalise une série d'essais au départ de divers substrats dans les conditions communes suivantes :
-   $H_5\{PMo_{10}V_2O_{40}\}$, 30-36$H_2O$ est utilisé comme catalyseur
-   le solvant est l'acétonitrile (6cm3 sauf indication contraire)

- la température est de 60°C et
- la pression partielle d'oxygène est de 1 atm.

Les conditions particulières et les résultats obtenus figurent dans le tableau III ci-après dans lequel les conventions suivantes sont utilisées :
- TT est le taux de transformation de la cétone considérée
- RR est le rendement du produit considéré

$$\left[ RR = \frac{\text{nombre de moles de produit formé}}{\text{nombre de moles de substrat initial}} \times 100 \right]$$

- Me représente un radical méthyle
- Ph représente un radical phényle
- i-Pr représente un radical isopropyle

- Q la quantité de catalyseur chargée

EP 0 355 075 B1

## TABLEAU III

| EX N° | SUBSTRAT | | | | | PRODUIT | |
|---|---|---|---|---|---|---|---|
| | NATURE | mmol | Q(mmol) | DUREE h | TT(%) | NATURE | RR(%) |
| 16 | Me, =O | 4,4 | 0,025 | 2 | 96 | $CH_3-C(O)-(CH_2)_3-COOH$ | 94 |
| 17 | Ph, =O | 2,4 | 0,015 | 6 | 96 | $Ph-C(O)-(CH_2)_4-COOH$ | 90 |
| 18 (*) | Me, Me, =O | 4,0 | 0,025 | 2 | 99 | $CH_3-C(O)-CH_2-CH(CH_3)-CH_2-COOH$ | 94 |
| 19 | Me, Me, =O | 11,0 | 0,075 | 6 | 91 | $CH_3-C(O)-(CH_2)_3-CH(CH_3)-COOH$ | 89 |
| 20 | i-Pr, Me, =O | 8,70 | 0,070 | 24 | 88 | $i-Pr-C(O)-(CH_2)_2-CH(CH_3)-CH_2-COOH$ | 84 |

(*) 2 cm3 d'acétonitrile

**Revendications**

1.  Procédé de préparation d'acides carboxyliques aliphatiques choisis parmi les diacides et les cétoacides aliphatiques, par oxydation de cétones monocyliques choisis parmi les composés dont le groupe carbonyle est lié directement à des atomes de carbone pour former un cycle unique saturé de 5 à 8 atomes de carbone, le cycle pouvant comporter en outre un ou deux substituants choisis parmi les radicaux alkyles en $C_1$-$C_4$ et le groupe phényle, au moyen d'oxygène moléculaire ou d'un gaz en contenant, caractérisé en ce que le catalyseur est choisi parmi les composés du vanadium répondant à l'une quelconque des formules (I) et (II) ci-après :

    $$H_{(3+n)} [PM_{12-n} V_n O_{40}], y\ H_2O \qquad (I)$$

    $$VO(Y)_m \qquad (II)$$

    dans lesquelles :
    -   n est un entier supérieur ou égal à 1 et inférieur ou égal à 6,
    -   M représente un atome de molybdène ou de tungstène,
    -   y est un entier, pouvant être nul, inférieur à 50,
    -   Y représente un groupe acétylacétonate ou un groupement alcoxy comportant de 1 à 10 atomes,
    -   m a pour valeur 2 ou 3,
    et en ce que la réaction est conduite en milieu liquide.

2.  Procédé selon la revendication 1, caractérisé en ce que le milieu liquide comprend un solvant ou diluant inerte dans les conditions réactionnelles, vis-à-vis des constituants du mélange réactionnel.

3.  Procédé selon la revendication 1 ou 2, caractérisé en ce que le solvant ou diluant utilisé est l'acétonitrile.

4.  Procédé selon l'une quelconque des revendications précédentes, caractérisé en ce que le catalyseur répond à la formule (I) donnée dans la revendication 1.

5.  Procédé selon la revendication 4, caractérisé en ce que le catalyseur répond à la formule (I) dans laquelle n vaut 1 ou 2 et y est un entier supérieur ou égal à 30 et inférieur ou égal à 40.

6.  Procédé selon la revendication 4 ou 5, caractérisé en ce que le catalyseur répond à la formule (I) dans laquelle M représente un atome de molybdène.

7.  Procédé selon l'une quelconque des revendications précédentes, caractérisé en ce que la température est comprise entre 25 et 120 °C et, de préférence entre 40 et 100 °C

8.  Procédé selon l'une quelconque des revendications précédentes, caractérisé en ce que la pression partielle d'oxygène est comprise entre 0,2 et 20 bars.

9.  Procédé selon l'une quelconque des revendications précédentes, caractérisé en ce que la concentration en vanadium dans le milieu réactionnel est comprise entre 0,001 et 0,5 mol.$l^{-1}$ et, de préférence, entre 0,005 et 0,1 mol.$l^{-1}$.

10. Procédé selon l'une quelconque des revendications précédentes, caractérisé en ce que la cétone monocyclique est la cyclohexanone.

11. Procédé selon l'une quelconque des revendications précédentes, caractérisé en ce que le milieu réactionnel renferme un alcool.

**Claims**

1.  Process for the preparation of aliphatic carboxylic acids chosen from aliphatic diacids and keto acids, by oxidation of monocyclic ketones chosen from compounds in which the carbonyl group is directly linked to carbon atoms to form single saturated ring containing 5 to 8 carbon atoms, the ring being able in addition to include one or two substituents chosen from $C_1$-$C_4$ alkyl radicals and the phenyl group, by means of molecular oxygen or of a gas in which it is contained, characterized in that the catalyst is chosen from the vanadium compounds corresponding to either of the formulae (I) and (II) below:

$$H_{(3+n)} [PM_{12-n} V_n O_{40}] \cdot y H_2 O \qquad (I)$$

$$VO(Y)_m \qquad (II)$$

in which:
- n is an integer greater than or equal to 1 and less than or equal to 6,
- M represents a molybdenum or tungsten atom,
- y is an integer, which may be zero, and is less than 50,
- Y represents an acetylacetonate group or an alkoxy group containing 1 to 10 atoms,
- m has the value 2 or 3,

and in that the reaction is carried out in a liquid medium.

2. Process according to claim 1, characterized in that the liquid medium comprises a solvent or diluent which is inert under the reaction conditions with respect to constituents of the reaction mixture.

3. Process according to claim 1 or 2, characterized in that the solvent or diluent used is acetonitrile.

4. Process according to any one of the preceding claims, characterized in that the catalyst corresponds to the formula (I) given in claim 1.

5. Process according to claim 4, characterized in that the catalyst corresponds to the formula (I) in which n equals 1 or 2 and y is an integer greater than or equal to 30 and less than or equal to 40.

6. Process according to claim 4 or 5, characterized in that catalyst corresponds to the formula (I) in which M represents a molybdenum atom.

7. Process according to any one of the preceding claims, characterized in that the temperature is between 25 and 120°C, and preferably between 40 and 100°C.

8. Process according to any one of the preceding claims, characterized in that the oxygen partial pressure is between 0.2 and 20 bars.

9. Process according to any one of the preceding claims, characterized in that the vanadium concentration in the reaction medium is between 0.001 and 0.5 $mol.l^{-1}$, and preferably between 0.005 and 0.1 $mol.l^{-1}$.

10. Process according to any one of the preceding claims, characterized in that the monocyclic ketone is cyclohexanone.

11. Process according to any one of the preceding claims, characterized in that the reaction medium contains an alcohol.

## Patentansprüche

1. Verfahren zur Herstellung aliphatischer Carbonsäuren, ausgewählt aus den aliphatischen Dicarbonsäuren und Ketocarbonsäuren, durch Oxidation monozy klischer Ketone, ausgewählt aus den Verbindungen, deren Carbonylgruppe direkt an die Kohlenstoffatome gebunden ist, so daß sie einen einzigen gesättigten Ring mit 5 bis 8 Kohlenstoffatomen bilden, wobei der Ring außerdem ein oder zwei Substituenten tragen kann, ausgewählt aus den Alkylresten mit $C_1$-$C_4$ und der Phenylgruppe, mit molekularem Sauerstoff oder ein ihn enthaltendes Gas, dadurch gekennzeichnet, daß der Katalysator aus den Vanadiumverbindungen ausgewählt ist, die einer beliebigen der nachstehenden Formeln (I) und (II) entspricht:

$$H_{(3+n)}[PM_{12-n}V_n O_{40}], y H_2 O \qquad (I)$$

VO(Y)$_m$     (II)

in denen:

- n ein ganze Zahl größer oder gleich 1 und kleiner oder gleich 6 ist,
- M für ein Molybdän- oder Wolframatom steht,
- Y eine ganze Zahl kleiner als 50 ist, die gleich Null sein kann,
- Y für eine Acetylacetonat- oder eine Alkoxygruppe mit 1 bis 10 Atomen steht,
- m den Wert 2 oder 3 hat,

und dadurch, daß die Reaktion im flüssigen Medium durchgeführt wird.

2. Verfahren nach Anspruch 1, dadurch gekennzeichnet, daß das flüssige Medium ein Lösungsmittel oder Verdünnungsmittel enthält, das unter den Reaktionsbedingungen inert gegenüber den Bestandteilen des Reaktionsgemischs ist.

3. Verfahren nach Anspruch 1 oder 2, dadurch gekennzeichnet, daß das verwendete Lösungsmittel oder Verdünnungsmittel Acetonitril ist.

4. Verfahren nach einem beliebigen der vorhergehenden Ansprüche, dadurch gekennzeichnet, daß der Katalysator der in Anspruch 1 angegebenen Formel (I) entspricht.

5. Verfahren nach Anspruch 4, dadurch gekennzeichnet, daß der Katalysator der Formel (I) entspricht, in der n den Wert 1 oder 2 hat und y eine ganze Zahl größer oder gleich 30 und kleiner oder gleich 40 ist.

6. Verfahren nach Anspruch 4 oder 5, dadurch gekennzeichnet, daß der Katalysator der Formel (I) entspricht, in der M für ein Molybdänatom steht.

7. Verfahren nach einem beliebigen der vorhergehenden Ansprüche, dadurch gekennzeichnet, daß die Temperatur zwischen 25 und 120°C und vorzugsweise zwischen 40 und 100°C liegt.

8. Verfahren nach einem beliebigen der vorhergehenden Ansprüche, dadurch gekennzeichnet, daß der Sauerstoffpartialdruck zwischen 0,2 und 20 bar liegt.

9. Verfahren nach einem beliebigen der vorhergehenden Ansprüche, dadurch gekennzeichnet, daß die Konzentration an Vanadium in dem Reaktionsmedium zwischen 0,001 und 0,5 mol•l$^{-1}$ und vorzugsweise zwischen 0,005 und 0,1 mol•l$^{-1}$ liegt.

10. Verfahren nach einem beliebigen der vorhergehenden Ansprüche, dadurch gekennzeichnet, daß das monozyklische Keton das Cyclohexanon ist.

11. Verfahren nach einem beliebigen der vorhergehenden Ansprüche, dadurch gekennzeichnet, daß das Reaktionsmedium einen Alkohol enthält.